# EUROPÄISCHE PATENTSCHRIFT

(11) **EP 0 421 218 B1**
(45) Veröffentlichungstag und Bekanntmachung des Hinweises auf die Patenterteilung: **08.06.1994**
(21) Anmeldenummer: 90118261.8
(22) Anmeldetag: 22.09.1990
(51) Int. Cl.: C07C 53/50, C07C 51/58

(54) **Verfahren zur Herstellung von Trifluormethylgruppen enthaltende Carbonsäurehalogenide**
Process for the preparation of trifluoromethyl groups containing carboxylic acid halide
Procédé de préparation d'halogénure d'acide carboxylique contenant groupes trifluorométhylène

(30) Priorität: 05.10.1989 DE 3933223
(43) Veröffentlichungstag der Anmeldung: 10.04.1991
(73) Patentinhaber: BAYER AG, 51368 Leverkusen (DE)
(72) Erfinder: Bielefeldt, Dietmar, Dr., D-4030 Ratingen 6 (DE); Ziemann, Heinz, Dr., D-5653 Leichlingen (DE)

(56) Entgegenhaltungen:
- EP-A- 0 070 463
- EP-A- 0 132 733
- FR-A- 2 225 484

## Beschreibung

Die vorliegende Erfindung betrifft ein neues Verfahren zur Herstellung von Trifluormethylgruppen enthaltenden Carbonsäurehalogeniden aus perhalogenierten Kohlenwasserstoffen, Olefinen und Kohlenmonoxid.

Trichlormethylgruppen enthaltende Carbonsäurehalogenide sind bekannt. Sie werden beispielsweise als Zwischenprodukte zur Herstellung von fluorierten Carbonsäuren und deren Salzen verwendet, bei denen es sich um Tenside und oberflächenaktive Mittel handelt. Sie können auch als Zwischenprodukte zur Herstellung von oleophoben, hydrophoben und schmutzabweisenden Mitteln zur Textilausrüstung benutzt werden (siehe US-PS 4 221 734 und Ullmann, Encyklopädie der technischen Chemie, Band 22, S. 500 (1982)).

Üblicherweise stellt man Trifluormethylgruppen enthaltende Verbindungen aus den entsprechenden Trichlormethylgruppen enthaltenden Verbindungen durch Chlor/Fluor-Austausch mit Flußsäure her. Nachteilig bei diesem Verfahren ist, daß man die Fluorierung individuell auf einer höheren Verarbeitungsstufe durchführen muß, was besonderen technischen Aufwand erfordert.

Es wurde nun ein Verfahren zur Herstellung von Trifluormethylgruppen enthaltenden Carbonsäurehalogeniden der Formel (I) gefunden 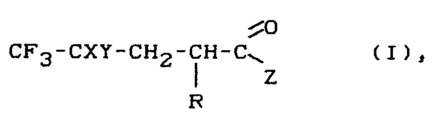
in der
- R: für Wasserstoff oder gesättigtes, ungesättigtes, geradkettiges oder verzweigtes C₁- bis C₁₀-Alkyl und
- Z: für Fluor, Chlor, Brom oder Jod stehen und
- X: für Fluor, Chlor, Brom, Jod oder Trifluormethyl und
- Y: für Fluor, Chlor, Brom oder Jod stehen,
wobei im Falle Z = Chlor X und Y nicht für Fluor stehen,
das dadurch gekennzeichnet ist, daß man ein Perhalogenalkan der Formel (II)

CF₃-CXYZ (II),

in der
X, Y und Z die bei Formel (I) angegebene Bedeutung haben,
mit Kohlenmonoxid und einem Olefin der Formel (III) umsetzt 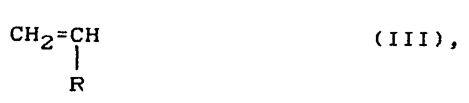
in der
- R: die bei Formel (I) angegebene Bedeutung hat,
und unter Zugabe eines ein Element der VI. bis VIII. Nebengruppe des Periodensystems der Elemente und Carbonylgruppen enthaltenden Katalysators arbeitet.

In den Formeln (II) und (III) stehen bevorzugt
- R: für Wasserstoff oder gesättigtes oder ungesättigtes, geradkettiges C₁- bis C₃-Alkyl und
- Z: für Chlor, Brom oder Jod und
- Y: für Fluor, Chlor oder Trifluormethyl und
- Y: für Fluor oder Chlor,
wobei im Falle Z = Chlor X und Y nicht für Fluor stehen.

Besonders bevorzugt sind die Kombinationen
- R =: Wasserstoff, X = Y = Z = Chlor,
- R =: Wasserstoff, Z = Brom, X = Y = Chlor,
- R =: Wasserstoff, Z = Jod, X = Y = Fluor,
- R =: Wasserstoff, Z = Jod, X = Trifluormethyl, Y = Fluor und
- R =: Methyl, X = Y = Z = Chlor.

Ganz besonders bevorzugt sind die Kombinationen
- R =: Wasserstoff, X = Y = Z = Chlor und
- R =: Wasserstoff, Z = Jod, X = Y = Fluor.

Bei den einzusetzenden Perhalogenalkanen der Formel (II) handelt es sich um Handelsprodukte oder auf einfache und bekannte Weise herstellbare Materialien.

Das benötigte Kohlenmonoxid kann in handelsüblichen Qualitäten eingesetzt werden.

Der benötigte Katalysator enthält zwingend ein Element der VI. bis VIII. Nebengruppe des Periodensystems der Elemente und Carbonylgruppen. Bevorzugte Elemente der VI. bis VIII. Nebengruppe sind Molybdän, Kobalt, Eisen, Ruthenium, Rhodium, Osmium und Iridium. Bei den Metallcarbonylen kann es sich um ein- oder mehrkernige Verbindungen handeln.

Gegebenenfalls können für das erfindungsgemäße Verfahren als Katalysatoren geeignete Metallcarbonyle zusätzlich zu Carbonylgruppen einen oder mehrere weitere Liganden enthalten, beispielsweise Wasserstoffatome, Halogenatome, Phosphortrialkyle, Phosphortriaryle und/oder Cyclopentadien.

Bevorzugt sind folgende Metallcarbonyle:

| | |
|---|---|
| Ni(CO)₄ | Rh₆(CO)₁₆ |
| Co₂ (CO)₈ | Co₂(CO)₆[P(C₄H₉)₃]₂ |
| Rh₄(CO)₁₂ | HRh(CO)(P(C₆H₅)₃]₃ |
| Ru₃(CO)₁₂ | Ir(CO)[P(C₆H₅)₃]₂Cl |
| Os₃(CO)₁₂ | Cp₂Fe₂(CO)₄ |
| Ir₄(CO)₁₂ | Cp₂Mo₂(CO)₆ |
| (Cp = Cyclopentadien). | |

Zusätzlich zu den oben beschriebenen Metallcarbonylen kann gegebenenfalls ein Co-Katalysator eingesetzt werden. Dabei kann es sich beispielsweise um ein Palladiumsalz, vorzugsweise um PdCl₂, oder um Zinn(II)-chlorid handeln.

Vorzugsweise setzt man sowohl Kohlenmonoxid als auch das Perhalogenalkan der Formel (II) in molarem Überschuß, bezogen auf das Olefin der Formel (III), ein. Beispielsweise kann man 3 oder mehr Mole Kohlenmonoxid und 3 oder mehr Mole des Perhalogenalkans der Formel (II) auf 1 Mol des Olefins der Formel (III) einsetzen.

Der Metallcarbonylkatalysator kann beispielsweise in einer Menge von 0,01 bis 100 mMol, bezogen auf 1 Mol des Olefins der Formel (III), eingesetzt werden. Vorzugsweise beträgt diese Menge 0,1 bis 10 mMol.

Der Co-Katalysator kann gegebenenfalls in einer Menge von beispielsweise 0 bis 100 mMol, bezogen auf 1 Mol des Olefins der Formel (III), eingesetzt werden.

Die Reaktionstemperatur kann in weiten Grenzen variiert werden. Vorzugsweise beginnt man mit der Reaktion bei relativ tiefen Temperaturen, z.B. bei -20 bis +50°C, erhöht im Laufe der Reaktion die Temperatur und führt sie beispielsweise bei 70 bis 200°C zu Ende.

Gegebenenfalls kann man in Gegenwart eines inerten Lösungsmittels arbeiten. Vorzugsweise wird jedoch kein Lösungsmittel eingesetzt.

Da die Ausgangs- und Reaktionsprodukte zum Teil bei Reaktionstemperaturen und Normaldruck gasförmig sind, wird das erfindungsgemäße Verfahren im allgemeinen unter Druck ausgeführt. Die Mengendosierung von Kohlenmonoxid und Olefin der Formel (III) kann dann durch Einstellung und gegebenenfalls Aufrechterhaltung von bestimmten (Partial-)Drucken im Reaktionsgefäß erfolgen. Die Reaktionsgefäße, z.B. Autoklaven, sollten im allgemeinen bei Drucken bis 300 bar betrieben werden können.

Die erfindungsgemäße Umsetzung ist im allgemeinen dann beendet, wenn kein Kohlenmonoxid und kein Olefin der Formel (III) mehr umgesetzt wird. Dies kann beispielsweise daran erkannt werden, daß sich der Druck im Reaktionsgefäß (bei konstanter Temperatur) nicht mehr ändert.

Das nach dem Ende der Reaktion vorliegende Reaktionsgemisch kann beispielsweise aufgearbeitet werden, indem man es abkühlt, entspannt und nacheinander überschüssige Ausgangsprodukte, gegebenenfalls verwendetes Lösungsmittel, das hergestellte Carbonsäurehalogenid der Formel (I) und gegebenenfalls Nebenprodukte abdestilliert. Es verbleibt dann im allgemeinen ein schwer flüchtiger Rückstand, der im wesentlichen den eingesetzten Katalysator enthält. Dieser kann gegebenenfalls für weitere Ansätze wieder verwendet werden.

Das erfindungsgemäße Verfahren zur Herstellung von Trifluormethylgruppen enthaltenden Carbonsäurehalogeniden hat den Vorteil, daß von Trifluormethylgruppen enthaltenden Handelsprodukten und einfach zugänglichen Perhalogenalkanen ausgegangen werden kann und eine individuelle Fluorierung auf einer höheren Verarbeitungsstufe vermieden werden kann. Außerdem ist es überraschend, daß die erfindungsgemäße 3-Komponenten-Reaktion (Perhalogenalkan + Olefin + Kohlenmonoxid) mit guten Ausbeuten abläuft, denn solche Reaktionen sind im allgemeinen wegen ihrer komplizierten Kinetik nicht sehr wahrscheinlich.

### Beispiele

### Beispiel 1

### Herstellung von CF₃-CCl₂-CH₂-CH₂-COCl

In einem 3 l-Edelstahlautoklaven wurden zu einer Lösung von 2 g PdCl₂ und 2,3 g Co₂(CO)₈ in 1000 ml (8,6 Mol) CF₃CCl₃ bei 23°C ein Gasgemisch von 20 bar Ethylen und 100 bar Kohlenmonoxid aufgedrückt. Das Gemisch wurde schrittweise auf 140°C aufgeheizt und der Druck durch Nachdrücken des Gasgemisches konstant gehalten. Das Ende der Reaktion wurde mit dem Ende der Gasaufnahme nach 15 Stunden erreicht. Nach dem Abkühlen und der Druckentspannung wurde das erhaltene Reaktionsgemisch fraktioniert destilliert. Die Ausbeute an 4,4,4-Trifluor-3,3-dichlor-n-buttersäurechlorid betrug 320 g. Der Siedepunkt betrug 91 bis 95°C bei 60 mbar. Das IR-Spektrum zeigte charakteristische Absorptionen bei 1800 cm⁻¹ (CO) und 1235, 1205 und 1190 cm⁻¹ (CF₃). Das Massenspektrum ergab einen Wert für m⁺/e von 242.

### Beispiel 2

### Herstellung von

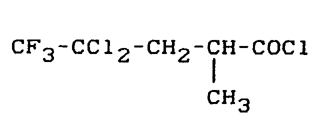

Es wurde analog Beispiel 1 verfahren, jedoch wurden statt 20 bar Ethylen 10 bar Propen aufgedrückt. Das Ende der Reaktion wurde nach 20 Stunden erreicht.

Die Ausbeute an 4,4,4-Trifluor-3,3-dichlor-1-methyl-n-buttersäurechlorid betrug 105 g. Der Siedepunkt lag bei 105 bis 107°C bei 150 mbar. Das IR-Spektrum wies eine charakteristische Absorption bei 1785 cm⁻¹ auf (CO).

## Patentansprüche

1. Verfahren zur Herstellung von Trifluormethylgruppen enthaltenden Carbonsäurehalogeniden der Formel (I) 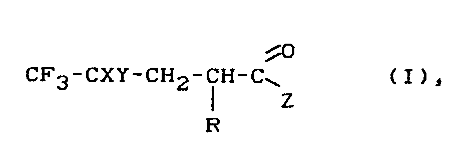 in der
R für Wasserstoff oder gesättigtes, ungesättigtes, geradkettiges oder verzweigtes C₁- bis C₁₀-Alkyl und
Z für Fluor, Chlor, Brom oder Jod stehen und
X für Fluor, Chlor, Brom, Jod oder Trifluormethyl und
Y für Fluor, Chlor, Brom oder Jod stehen,
wobei im Falle Z = Chlor X und Y nicht für Fluor stehen,
dadurch gekennzeichnet, daß man ein Perhalogenalkan der Formel (II)
CF₃-CXYZ (II),
in der
X, Y und Z die bei Formel (I) angegebene Bedeutung haben,
mit Kohlenmonoxid und einem Olefin der Formel (III) umsetzt 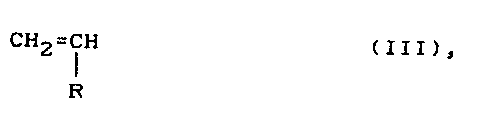 in der
R die bei Formel (I) angegebene Bedeutung hat,
und unter Zugabe eines ein Element der VI. bis VIII. Nebengruppe des Periodensystems der Elemente und Carbonylgruppen enthaltenden Katalysators arbeitet.

2. Verfahren nach Anspruch 1, dadurch gekennzeichnet, daß der Katalysator Molybdän, Kobalt, Eisen, Ruthenium, Rhodium, Osmium oder Iridium enthält.

3. Verfahren nach Ansprüchen 1 und 2, dadurch gekennzeichnet, daß es sich bei dem Katalysator um
| | |
|---|---|
| Ni(CO)₄ | Rh₆(CO)₁₆, |
| Co₂ (CO)₈ | Co₂(CO)₆[P(C₄H₉)₃]₂, |
| Rh₄(CO)₁₂ | HRh(CO)(P(C₆H₅)₃]₃, |
| Ru₃(CO)₁₂ | Ir(CO)[P(C₆H₅)₃]₂Cl, |
| Os₃(CO)₁₂ | Cp₂Fe₂(CO)₄ oder |
| Ir₄(CO)₁₂ | Cp₂Mo₂(CO)₆ |
| (Cp = Cyclopentadien) handelt. | |

4. Verfahren nach Ansprüchen 1 bis 3, dadurch gekennzeichnet, daß ein Palladiumsalz oder Zinn(II)-chlorid als Co-Katalysator eingesetzt wird.

5. Verfahren nach Ansprüchen 1 bis 4, dadurch gekennzeichnet, daß man Kohlenmonoxid und das Perhalogenalkan der Formel (II) in molarem Überschuß bezogen auf das Olefin der Formel (III) einsetzt`

## Claims

1. Process for the preparation of carboxylic acid halides containing trifluoromethyl groups, of the formula (I) 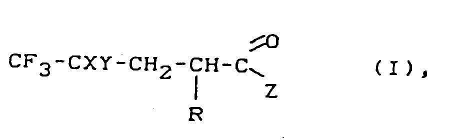 in which
R represents hydrogen or saturated or unsaturated, straight-chain or branched C₁- to C₁₀-alkyl,
Z represents fluorine, chlorine, bromine or iodine,
X represents fluorine, chlorine, bromine, iodine or trifluoromethyl and
Y represents fluorine, chlorine, bromine or iodine,
and in the case where Z = chlorine, X and Y do not represent fluorine,
characterized in that a perhalogenoalkane of the formula (II)
CF₃-CXYZ (II)
in which
X, Y and Z have the meaning given in the case of formula (I),
is reacted with carbon monoxide and an olefin of the formula (III) 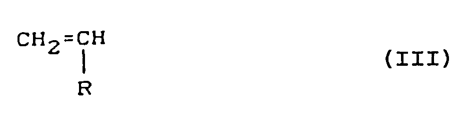 in which
R has the meaning given in the case of formula (I),
with the addition of a catalyst containing an element of sub-group VI to VIII of the Periodic Table of the Elements and carbonyl groups.

2. Process according to Claim 1, characterized in that the catalyst contains molybdenum, cobalt, iron, ruthenium, rhodium, osmium or iridium.

3. Process according to Claims 1 and 2, characterized in that the catalyst is
| | |
|---|---|
| Ni(CO)₄ | Rh₆(CO)₁₆ |
| Co₂(CO)₈ | Co₂(CO)₆[P(C₄H₉)₃]₂ |
| Rh₄(CO)₁₂ | HRh(CO)[P(C₆H₅)₃]₃ |
| Ru₃(CO)₁₂ | Ir(CO)[P(C₆H₅)₃]₂Cl |
| Os₃(CO)₁₂ | Cp₂Fe₂(CO)₄ or |
| Ir₄(CO)₁₂ | Cp₂Mo₂(CO)₆ |
| (Cp = cyclopentadiene). | |

4. Process according to Claims 1 to 3, characterized in that a palladium salt or tin(II) chloride is employed as a co-catalyst.

5. Process according to Claims 1 to 4, characterized in that carbon moxide and the perhalogenoalkane of the formula (II) are employed in a molar excess relative to the olefin of the formula (III).

## Revendications

1. Procédé de production d'halogénures d'acides carboxyliques contenant des groupes trifluorométhyle, de formule (I) 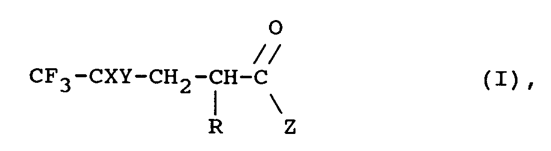 dans laquelle
R représente de l'hydrogène ou un groupe alkyle en C₁ à C₁₀ saturé ou non saturé à chaîne droite ou ramifiée,
Z représente du fluor, du chlore, du brome ou de l'iode,
X est du fluor, du chlore, du brome, de l'iode ou un groupe trifluorométhyle et
Y représente du fluor, du chlore, du brome ou de l'iode,
X et Y n'étant pas du fluor au cas ou Z représente du chlore,
caractérisé en ce qu'on fait réagir un alcane perhalogéné de formule (II)
CF₃-CXYZ (II),
dans laquelle
X, Y et Z ont la définition indiquée pour la formule (I)
avec de l'oxyde de carbone et une oléfine de formule (III) 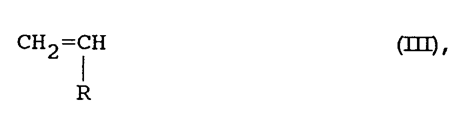 dans laquelle
R a la définition indiquée pour la formule (I)
et on opère en ajoutant un catalyseur contenant un élément d'un sous-groupe VI à VIII du Système Périodique des Eléments et des groupes carbonyle.

2. Procédé suivant la revendication 1, caractérisé en ce que le catalyseur contient du molybdène, du cobalt, du fer, du ruthénium, du rhodium, de l'osmium ou de l'iridium.

3. Procédé suivant les revendications 1 et 2, caractérisé en ce que le catalyseur est :
| | |
|---|---|
| Ni(CO)₄ | Rh₆(CO)₁₆, |
| Co₂(CO)₈ | Co₂(CO)₆[P(C₄H₉)₃]₂, |
| Rh₄(CO)₁₂ | HRh(CO)[P(C₆H₅)₃]₃, |
| Ru₃(CO)₁₂ | Ir(CO)[P(C₆H₅)₃]₂Cl, |
| Os₃(CO)₁₂ | Cp₂Fe₂(CO)₄ ou |
| Ir₄(CO)₁₂ | Cp₂Mo₂(CO)₆ |
| (Cp = cyclopentadiène) | |

4. Procédé suivant les revendications 1 à 3, caractérisé en ce qu'on utilise un sel de palladium ou du chlorure d'étain (II) comme cocatalyseur.

5. Procédé suivant les revendications 1 à 4, caractérisé en ce qu'on utilise de l'oxyde de carbone et l'alcane perhalogéné de formule (II) en excès molaire par rapport à l'oléfine de formule (III).
